# FASCICULE DE BREVET EUROPEEN

(11) **EP 1 977 244 B1**
(45) Date de publication et mention de la délivrance du brevet: **21.07.2010**
(21) Numéro de dépôt: 06841938.1
(22) Date de dépôt: 14.12.2006
(51) Int. Cl.: G01N 33/569

(54) **DISTINCTION DES MENINGITES BACTERIENNES ET VIRALES**
UNTERSCHEIDUNG ZWISCHEN BAKTIERELLER MENINGITIS UND VIRALER MENINGITIS
DISTINCTION BETWEEN BACTERIAL MENINGITIS AND VIRAL MENINGITIS

(30) Priorité: 14.12.2005 FR 0512687
(43) Date de publication de la demande: 08.10.2008
(73) Titulaire: Assistance Publique - Hôpitaux de Paris, 75004 Paris (FR)
(72) Inventeur: CHALUMEAU, Martin, F-75014 Paris (FR); DUBOS, François, F-59000 Lille (FR); GENDREL, Dominique, F-75014 Paris (FR); BREART, Gérard, F-92210 Saint-Cloud (FR)
(74) Mandataire: Novagraaf Technologies
(86) Numéro de dépôt international: PCT/FR2006/002734
(87) Numéro de publication internationale: WO 2007/068830

(56) Documents cités:
- JEREB M ET AL: "Predictive value of serum and cerebrospinal fluid procalcitonin levels for the diagnosis of bacterial meningitis" INFECTION, vol. 29, no. 4, août 2001 (2001-08), pages 209-212, XP002379559 ISSN: 0300-8126
- FERRIERE F: "Procalcitonin: A new marker for bacterial infections" ANNALES DE BIOLOGIE CLINIQUE, vol. 58, no. 1, janvier 2000 (2000-01), pages 49-59, XP002379558 ISSN: 0003-3898
- MARY R ET AL: "[Acute meningitidis, acute phase proteins and procalcitonin.]" ANNALES DE BIOLOGIE CLINIQUE, vol. 61, no. 2, 2003, pages 127-137, XP002379560 ISSN: 0003-3898
- BENDER ANDREAS ET AL: "Severe symptomatic aseptic chemical meningitis following myelography - The role of procalcitonin" NEUROLOGY, vol. 63, no. 7, octobre 2004 (2004-10), pages 1311-1313, XP002379561 ISSN: 0028-3878
- GENDREL D: "Apport des donnees biochimiques dans le diagnostic des meningites purulentes communautaires" MEDECINE ET MALADIES INFECTIEUSES, SOCIETE FRANCAISE D'EDITIONS MEDICALES, PARIS, FR, vol. 26, décembre 1996 (1996-12), pages 1068-1072, XP004907301 ISSN: 0399-077X
- GENDREL ET AL: "Infection urinaire et marqueurs biologiques: proteine C reactive, interleukines et procalcitonine" ARCHIVES DE PEDIATRIE, ELSEVIER, PARIS, FR, vol. 5, 1998, pages 269S-273S, XP005033143 ISSN: 0929-693X
- VAN ROSSUM A ET AL: "Procalcitonin as an early marker of infection in neonates and children" LANCET INFECTIOUS DISEASES, US, vol. 4, no. 10, octobre 2004 (2004-10), pages 620-630, XP004808552 ISSN: 1473-3099
- SCHWARZ ET AL.: "Serum procalcitonin levels in bacterial and abacterial meningitis." CRITICAL CARE MEDICINE, vol. 28, no. 6, 2000, page 18281832, XP008113032
- VIALLON ET AL.: "Diagnostic rapide du type de ménangite (bactérienne ou virale) par le dosage de la procalcitonine sérique" PRESSE MEDICALE, vol. 29, no. 11, 2000, pages 584-588, XP008113031
- GENDREL ET AL.: "Procalcitonine, protéine C-réactive et interleukine 6 dans les méningites bactériennes et virales de l'enfant." PRESSE MÉDICALE, vol. 27, no. 23, 1998, pages 1135-1139, XP008113033
- SCHWARZ ET AL.: "Serum procalcitonin levels in bacterial and abacterial meningitis." CRITICAL CARE MEDICINE, vol. 28, no. 6, 2000, page 18281832, XP008113032
- VIALLON ET AL.: "Diagnostic rapide du type de ménangite (bactérienne ou virale) par le dosage de la procalcitonine sérique" PRESSE MEDICALE, vol. 29, no. 11, 2000, pages 584-588, XP008113031
- GENDREL ET AL.: "Procalcitonine, protéine C-réactive et interleukine 6 dans les méningites bactériennes et virales de l'enfant." PRESSE MÉDICALE, vol. 27, no. 23, 1998, pages 1135-1139, XP008113033

## Description

La présente invention se rapporte au domaine de la distinction entre méningites bactériennes et virales. Elle concerne tout particulièrement un procédé de détection d'une infection du type méningite bactérienne consistant notamment en la détermination de paramètres biologiques comme la concentration en procalcitonine dans le sang et la concentration en protéines présentes dans le liquide céphalo-rachidien.

Les méningites aiguës sont des infections communes, majoritairement d'origine virale (82-94%) et qui peuvent alors se résorber spontanément. Toutefois, lorsqu'elles sont d'origine bactérienne (6-18%), les méningites peuvent être mortelles et sont fréquemment associées à des séquelles neurologiques sévères, particulièrement lorsque le diagnostique et le traitement ont été initiés tardivement. À l'heure actuelle, en raison de la difficulté à distinguer rapidement et de manière certaine les méningites bactériennes des méningites virales, les patients chez lesquels une méningite est identifiée sont traités rapidement avec des antibiotiques en hospitalisation et leur traitement est prolongé jusqu'à ce que l'origine de la méningite soit clairement diagnostiquée. Les conséquences de cette administration systématique d'antibiotiques aux patients chez lesquels une méningite est identifiée sont importantes non seulement au niveau économique mais aussi au niveau médical. En effet, ce type d'administration contribue au risque de développement de souches bactériennes résistantes aux antibiotiques, de maladies nosocomiales et surtout présente un coût élevé. Une étude menée aux Etats-Unis a ainsi montré que l'hospitalisation des patients atteints de méningite virale représentait un coût de 250 à 300 millions de dollars par an (Khetsuriani et al., Neuroepidemiology 2003, vol. 22, pages 345-352).

En conséquence, il apparaît nécessaire d'établir un diagnostic rapide et simple permettant de distinguer les méningites bactériennes des méningites virales avec une sensibilité de 100% pour les méningites bactériennes.

De nombreux signes cliniques sont connus pour distinguer les méningites bactériennes des méningites virales avec une certaine sensibilité comme décrit dans les articles de Michelow et al. (Pediatr. Infect. Dis. Journal, 2000, vol. 19, pages 66-72) et Tatara et al. (Pediatr. Int., 2000, vol.42, pages 541-546). À titre d'exemples de tels signes cliniques, on peut citer le purpura, l'aspect dit " septique et/ou toxique " et les convulsions qui contribuent au diagnostic de la méningite bactérienne. Toutefois, ces signes cliniques ne sont pas toujours présents et détectables chez les patients atteints de méningite bactérienne.

Il existe également des tests bactériologiques susceptibles de donner un résultat avec une grande spécificité, mais ces tests n'atteignent pas la sensibilité requise de 100% pour les méningites bactériennes. Il existe donc toujours un risque avec de tels tests de ne pas identifier la totalité des patients atteints de méningites bactériennes. De tels tests ne peuvent donc pas être utilisés pour éviter l'administration systématique d'antibiotiques aux patients chez lesquels une méningite a été identifiée. À titre d'exemples de tests bactériologiques, on peut citer la coloration de Gram ou les études d'antigènes bactériens du liquide céphalo-rachidien comme décrit dans les publications de Nigrovic et al. (Pediatrics, 2002, vol. 110, pages 712-719), Saez-Llorens et al. (Lancet, 2003, vol. 88, pages 615-620), Tunkel et al. (Clin. Infect. Dis., 2004, vol. 39, pages 1267-1284), Maxson et al. (J. Pediatr., 1994, vol. 125, pages 235-238). Des marqueurs biologiques ont également été proposés pour améliorer le diagnostic étiologique des méningites, comme décrit dans les publications de Seaz-Lorens et al. (Lancet, 2003, vol. 88, pages 615-620) et Tunkel et al. (Clin. Infect. Dis., 2004, vol. 39, pages 1267-1284). À titre d'exemples de marqueurs biologiques proposés, on peut citer des marqueurs présents dans le sang comme la protéine C-réactive, l'interleukine 6, les leucocytes dont les neutrophiles, des marqueurs présents dans le liquide céphalo-rachidien comme les protéines, les leucocytes dont les neutrophiles et des marqueurs présents dans le sang et le liquide céphalo-rachidien comme la procalcitonine, le lactate, le glucose, tels que décrit dans Ferriere et al. (annales de biologie clinique, 2000, vol.58, pages 49-59), Jereb et al. (infection, 2001, vol. 29, pages 209-212), Mary et al. (annales de biologie clinique, 2003, vol.61, pages 127-137), Bender et al. (neurology, vol. 63, pages 1311-1313), Gendrel et al. (Méd. Mal. Infect., vol. 26, pages 1068-1072), Van Rossum et al., (Lancet Infect. Dis., 2004, vol. 4, pages 620-630), Schwartz et al. (critical care medicine, 2000, vol. 28, pages 1828-1832), Viallon et al. (presse médicale, 2000, vol. 29, pages 584-588), Gendrel et al., (presse médicale, 1998, vol. 27, pages 1133-1139).

De nombreuses règles combinant ces signes cliniques et/ou ces marqueurs biologiques ont été proposées afin de distinguer les méningites bactériennes et virales.

Ainsi, on peut citer la règle de Jaeger et al. (Eur. J. Clin. Microbiol. Infect. Dis. 200, vol. 19, pages 1418-1421) qui est un modèle basé sur la détermination de la concentration en glucose et en globules blancs dans le sang et la concentration en neutrophiles et en protéines dans le liquide céphalo-rachidien.

La règle de Bonsu et al. (Pediatr. Infect. Dis. J., 2004, vol. 23, pages 511-517) est une équation fractionnelle polynomiale basée sur la détermination de la concentration en neutrophiles et en protéines dans le liquide céphalo-rachidien et prend aussi en considération l'âge du patient.

La règle de Freedman et al. (Freedman et al., (Arch. Pediatr. Adolesc. Med., 2001, vol. 155, pages 1301-1306) est basée sur une liste de différentes caractéristiques incluant l'âge du patient, la coloration de Gram du liquide céphalo-rachidien et la concentration en globules blancs, en protéines, en glucose dans le liquide céphalo-rachidien.

La règle de Nigrovic et al. (Pediatrics 2002, vol. 110, pages 712-719) est également basée sur une liste de caractéristiques incluant la présence de convulsions chez le patient, la concentration en neutrophiles dans le sang, la coloration de Gram du liquide céphalo-rachidien, la concentration en neutrophiles et en protéines dans le liquide céphalo-rachidien.

La règle de Ooestenbrink et al. (Arch. Pediatr. Adolesc. Med., 2002, vol. 156, pages 1189-1194) associe dans une grille complexe des éléments cliniques avec des éléments biochimiques.

Toutefois, à l'heure actuelle il n'existe pas de tests rapides et simples permettant de distinguer les méningites bactériennes et virales avec la sensibilité requise de 100% pour les méningites bactériennes et avec une spécificité suffisamment élevée.

Les inventeurs ont maintenant établi un procédé rapide et simple de détection précoce de la présence d'une méningite bactérienne présentant une sensibilité de 100 % pour les méningites bactériennes et ceci avec une spécificité supérieure à 50 %.

Ainsi, un premier objet de l'invention correspond à un procédé *in vitro* de détection de la présence d'une méningite bactérienne consistant en :
i) la détermination de la concentration de procalcitonine présente dans un échantillon sanguin test ; et
ii) la comparaison de la concentration ainsi déterminée à la concentration de procalcitonine présente dans un échantillon de référence ou à une valeur de référence.
iii) la détermination de la concentration de protéines présentes dans un échantillon de liquide céphalorachidien test ; et
iv) la comparaison de la concentration ainsi déterminée à la concentration de protéines dans un échantillon de référence ou à une valeur de référence.

On entend par méningite bactérienne, une inflammation des méninges qui peut être provoquée par divers types de germes mais principalement trois : les méningocoques *(Neisseria meningitidis),* les pneumocoques *(Streptococcus pneumoniae)* et les *Haemophilus influenzae* de type b.

On entend par procalcitonine, la protéine précurseur de la calcitonine de 116 acides aminés monocaténaire décrite par Le Moullec JM *et al.* (SEQ ID NO :8 du brevet US 6,905,687, FEBS Letter, 1984, pages 167-193) et couramment désignée ProCT ou PCT. Bien que retrouvée dans différents tissus, à procalcitonine est synthétisée majoritairement dans le foie. Des expériences menées chez l'animal et l'homme, laissent supposer que la procalcitonine est impliquée dans la réaction inflammatoire, sans que son rôle y soit aujourd'hui clairement établi. La procalcitonine a été décrite comme un marqueur précoce des infections bactériennes sévères (pyélonéphrite) (Gendrel et al., archives de pédiatrie, Elsevier, Paris, 1998, vol. 5, pages 269S-273S).

On entend par échantillon sanguin test, un échantillon sanguin d'un individu susceptible d'être atteint de méningite bactérienne. Les échantillons sanguins peuvent être obtenus selon des techniques bien connues de l'Homme du Métier à l'aide par exemple d'une aiguille munie d'une seringue introduite dans une veine de l'avant-bras ou d'un pli du coude d'un individu. Un échantillon de 1 à 3 mL de sang obtenu chez un enfant pourra être suffisant pour mettre en oeuvre le procédé selon la présente invention. Avantageusement, l'échantillon de sang peut être traité afin d'inhiber les propriétés bactéricides normales du sang et les agents antimicrobiens éventuels par la dilution du sang et l'addition d'inhibiteurs tels que le polyanétholsulfonate de sodium (SPS) à la concentration de 0,025%.

Avantageusement, l'échantillon sanguin test correspond à un échantillon de sérum ou de plasma d'un individu dont on veut déterminer le statut au regard de la méningite bactérienne. Un tel échantillon de sérum ou de plasma peut être obtenu simplement par l'homme du métier par centrifugation d'un échantillon sanguin et récupération du surnageant.

On entend par échantillon de référence de l'étape ii), tout échantillon dont la concentration de procalcitonine après comparaison avec la concentration de procalcitonine dudit échantillon sanguin test offre une indication de la présence d'une méningite bactérienne chez ledit individu dont provient ledit échantillon sanguin test.

En effet, les inventeurs ont démontré que des individus atteints de méningite bactérienne présentent une concentration de procalcitonine dans le sang augmentée par rapport à des individus sains ou atteints d'une méningite virale. Ainsi, les inventeurs ont pu déterminer qu'une concentration de procalcitonine supérieure ou égale à 0,5 ng/ml dans l'échantillon sanguin test permet de conclure à une infection de l'individu testé pour une méningite bactérienne. À titre d'exemple de valeur de référence, on peut donc citer une concentration de procalcitonine de 0,5 ng/ml.

À titre d'exemples, d'échantillon de référence de l'étape ii), on peut citer des échantillons sanguins provenant d'un individu sain ou provenant d'un individu atteint d'une méningite virale, des échantillons de sérum sanguin provenant d'un individu sain ou provenant d'un individu atteint d'une méningite virale, des échantillons de plasma sanguin provenant d'un individu sain ou provenant d'un individu atteint d'une méningite virale ou encore une solution de procalcitonine à une concentration déterminée.

On entend par individu sain, un individu qui ne présente pas de pathologies.

On entend par méningite virale, une inflammation des méninges qui peut être provoquée par différents virus comme les entérovirus tels que Echovirus, Coxsackie, et plus rarement les virus du groupe herpès tels que les herpès 1 et 2, le Cytomégalovirus le virus d'Epstein-Barr, les virus varicelle zona et le virus HHV6 et plus rarement les arboviroses.

Selon un mode de réalisation préféré dudit procédé selon l'invention, ledit échantillon de référence de l'étape ii) est une solution de procalcitonine à une concentration déterminée.

On entend par solution de procalcitonine à une concentration déterminée, une solution de procalcitonine à une concentration comprise entre 0,05 ng/mL et 10 ng/mL, de préférence entre 0,1 ng/mL et 1 ng/mL et préférentiellement de 0,5 ng/ml. Une telle solution peut être obtenue simplement par l'homme du métier par la dissolution d'une quantité déterminée de procalcitonine purifiée et/ou recombinante dans un volume d'eau ou de solution tampon, comme le PBS. Une telle protéine purifiée et/ou recombinante peut notamment être obtenue selon les techniques décrites dans le brevet US 6,905,687.

La détermination de la concentration de procalcitonine présente dans un échantillon sanguin test peut s'effectuer selon des techniques bien connues de l'Homme du métier. À titre d'exemple, on peut citer les techniques immunologiques quantitatives utilisant des anticorps ou des fragments d'anticorps qui lient spécifiquement la procalcitonine telles que la technique ELISA ou encore les techniques décrites dans la Demande PCT WO 97/20213.

Selon un deuxième mode de réalisation préféré dudit procédé selon l'invention, la détermination de la concentration en procalcitonine présente dans un échantillon sanguin test comprend la mise en contact de l'échantillon avec un anticorps qui lie spécifiquement la procalcitonine.

La détermination de la concentration en procalcitonine à l'aide d'un anticorps qui lie spécifiquement la procalcitonine peut s'effectuer selon des techniques bien connues de l'Homme du métier comme par exemple par des techniques immunologiques quantitatives telles que la technique d'ELISA, les techniques décrites dans la Demande PCT WO 97/20213, la technique décrite dans Guillani et al. (Cancer Res., 1989, vol. 49, pages 6845-6851) et les méthodes mises en oeuvre dans les ensembles LUMItest® également désigné B.R.A.H.M.S. PCT LIA, B.R.A.H.M.S. PCT KRYPTOR® et LIAISON B.R.A.H.M.S. PCT disponibles chez B.R.A.H.M.S. Diagnostica (Berlin, Allemagne). Lesdites techniques immunologiques quantitatives peuvent mettent en oeuvre des anticorps monoclonaux qui lient spécifiquement la procalcitonine marqués directement ou indirectement à l'aide d'un second anticorps marqué par exemple par une enzyme comme la peroxydase, la phosphatase alcaline et la β-galactosidase, par un réactif luminescent comme la fluoresceine, la rhodamine, la cyanine ou à l'aide d'un second anticorps biotinylé.

Les anticorps ou les fragments d'anticorps qui lient spécifiquement la procalcitonine peuvent être des anticorps polyclonaux ou monoclonaux. Les fragments d'anticorps qui lient spécifiquement la procalcitonine peuvent être choisis dans le groupe comprenant les fragments Fab, F(ab')2, FV et sFv. À titre d'exemples d'anticorps monoclonaux qui lient spécifiquement la procalcitonine, on peut citer les anticorps décrits dans les brevets US 6,451,311, US 5,330,909 et US 6,133,427, les anticorps disponibles chez ABCAM avec les références « ab14813 », « ab11498 », « ab11494 », « ab14817 », « ab14816 », « ab24454 », l'anticorps disponible chez CHEMICON avec la référence « MAB3490 » et les anticorps disponibles chez GeneTex®, Inc. avec les références « GTX14813 », « GTX11498 », « GTX11494 », « GTX14817 » et « GTX14816 ».

On entend par protéines du liquide céphalo-rachidien, les protéines présentes dans le liquide contenu dans les espaces délimités par les méninges et dans les ventricules du cerveau. À titre d'exemples de protéines du liquide céphalo-rachidien, on peut citer la préalbumine, l'albumine, l'α1-globuline, l'α2-globuline, la β1-globuline et la β2-globuline et les γ-globulines.

On entend par échantillon de liquide céphalo-rachidien test, un échantillon de liquide céphalo-rachidien d'un individu susceptible d'être atteint de la méningite bactérienne. Dans le procédé selon l'invention, l'échantillon sanguin test et l'échantillon de liquide céphalo-rachidien test proviennent d'un même individu. Les échantillons de liquide céphalorachidien peuvent être obtenus selon des techniques bien connues de l'Homme du Métier par exemple par une ponction lombaire.

On entend par échantillon de référence de l'étape iv), tout échantillon dont la concentration de protéines après comparaison avec la concentration de protéines dudit échantillon de liquide céphalorachidien test offre une indication de la présence d'une méningite bactérienne chez ledit individu dont provient ledit échantillon de liquide céphalorachidien test.

En effet, les inventeurs ont démontré que des individus atteints de méningite bactérienne présentent une concentration en protéines dans le liquide céphalo-rachidien augmentée par rapport à des individus sains ou atteints d'une méningite virale. Ainsi, les inventeurs ont pu déterminer qu'une concentration de protéines dans le liquide céphalo-rachidien supérieure ou égale à 0,5 g/L dans l'échantillon de liquide céphalo-rachidien test permet de conclure à une infection de l'individu testé par une méningite bactérienne. À titre d'exemple de valeur de référence, on peut donc citer une concentration de protéines dans le liquide céphalo-rachidien de 0,5 g/L.

Le procédé selon l'invention, en identifiant les individus dont la concentration en procalcitonine est supérieure ou égale à 0,5 ng/ml et/ou dont la concentration en protéines dans le liquide céphalo-rachidien est supérieure ou égale à 0,5 g/L, permet d'identifier les individus atteints d'une méningite bactérienne avec une sensibilité de 100%.

À titre d'exemples, d'échantillon de référence de l'étape iv), on peut citer des échantillons de liquide céphalo-rachidien provenant d'un individu sain ou provenant d'un individu atteint d'une méningite virale ou une solution de protéines à une concentration déterminée.

Selon un troisième mode de réalisation préféré dudit procédé selon l'invention, ledit échantillon de référence de l'étape iv) peut correspondre à une solution de protéines à une concentration déterminée.

On entend par solution de protéines à une concentration déterminée, une solution de protéines à une concentration comprise entre 0,05 et 5 g/L, de préférence entre 0,1 et 1 g/L et de manière particulièrement préférée de 0,5 g/L. Une telle solution peut être obtenue simplement par l'homme du métier par la dissolution d'une quantité déterminée de protéines, notamment de la BSA (Bovine Serum Albumine) à la concentration voulue dans un volume d'eau ou de solution tampon comme le PBS.

La détermination de la concentration de protéines présentes dans un échantillon de liquide céphalo-rachidien test peut s'effectuer selon des techniques bien connues de l'Homme du Métier, comme des techniques immunologiques utilisant des anticorps ou des fragments d'anticorps qui lient spécifiquement les protéines du liquide céphalo-rachidien, des techniques basées sur la mesure de l'activité enzymatique desdites protéines du liquide céphalo-rachidien, des tests de biologie moléculaire, des tests physiques, des tests chimiques comme des tests colorimétriques, la détermination de la masse desdites protéines du liquide céphalo-rachidien comme la spectroscopie de masse.

Selon un quatrième mode de réalisation préféré dudit procédé selon l'invention, l'étape iii) de détermination de la concentration en protéines dans le liquide céphalo-rachidien est effectuée par un test colorimétrique.

Un tel test colorimétrique comprend la mise en contact dudit échantillon de liquide céphalo-rachidien avec un réactif colorimétrique dont la réaction avec lesdites protéines permet de déterminer leur concentration. À titre d'exemples de tests colorimétriques, on peut citer les méthodes de coloration au méthanol acétique, à l'amidoschwartz ou au rouge de pyrogallol utilisées par exemple dans le kit disponible chez ROCHE DIAGNOSTICS avec la référence « 03515826 ».

Avantageusement, ledit individu dont proviennent ledit échantillon sanguin test et éventuellement ledit échantillon de liquide céphalo-rachidien test à un âge compris entre 2 semaines et 25 ans, de préférence entre 3 semaines et 18 ans et tout préférentiellement entre 1 mois et 16 ans.

Avantageusement encore, le procédé selon l'invention est effectué sur des échantillons tests provenant d'un individu dont il est établi qu'il est atteint d'une méningite, laquelle est donc potentiellement d'origine bactérienne.

À titre d'exemple de tels individus, on peut citer des individus présentant plus de 5 globules blancs/mm³, de préférence plus de 7 globules blancs/mm³ dans leur liquide céphalo-rachidien tel que décrit dans les publications de Greenlee et al. (Dis. Clin. North. Am., 1990, vol.4, pages 583-598) et Saez-Lorens et al. (Dis. Clin. North. Am., 1990, vol.4, pages 623-644). La détermination de la concentration en globules blancs présents dans le liquide céphalo-rachidien peut être effectuée par des techniques bien connues de l'Homme du métier.

Avantageusement encore, le procédé selon l'invention n'est pas effectué sur des échantillons tests sanguins et éventuellement de liquide céphalo-rachidien provenant d'individus dont la probabilité d'atteinte par la méningite bactérienne est telle, que la mise en oeuvre dudit procédé selon l'invention est inutile.

À titre d'exemple de tels individus, on peut citer les individus ayant subi préalablement une intervention neurochirurgicale, les individus souffrant d'immunodépression et/ou les individus présentant un purpura, un aspect septique et/ou toxique et/ou des convulsions.

On entend par immunodépression, une diminution ou une suppression des réactions immunitaires d'un organisme.

On entend par purpura des taches hémorragiques provoquées par du sang extravasé en dehors des capillaires de la peau ou des muqueuses pouvant se traduire par des pétéchies ou une ecchymose. Ces taches sont de couleur rouge vif ou bleuâtre et ne s'effacent pas à la pression.

On entend par aspect septique et/ou toxique, un individu présentant une apparence léthargique, des signes de faible perfusion comme une couleur de peau pâle, cendrée tel que décrit dans la publication de Baker et al. (Pediatrics, 1990, vol. 85, pages 1040-1043) des signes d'hypoventilation ou d'hyperventilation ou de cyanose tels que décrit dans la publication de Baraff et al. (Pediatrics, 1993, vol. 2, pages 1-12).

On entend par convulsions, des contractions involontaires et instantanées, déterminant des mouvements localisés à un ou plusieurs groupes musculaires ou généralisés à tout le corps.

Avantageusement encore, le procédé selon l'invention n'est pas effectué sur des échantillons dont la qualité ne permet pas la mise en oeuvre du procédé selon l'invention.

À titre d'exemple de tels échantillons, on peut citer des échantillons de liquide céphalo-rachidien dont le prélèvement a entraîné une hémorragie localisée du patient. De tels échantillons de liquide céphalo-rachidien présentent alors une concentration de globules rouges supérieure à 12 000 globules rouges/mm³, de préférence supérieure à 10 000 globules rouges/mm³. La détermination de la concentration en globules rouges présents dans l'échantillon de liquide céphalo-rachidien peut être effectuée par des techniques bien connues de l'Homme du Métier.

Avantageusement encore, le procédé selon l'invention n'est pas mis en oeuvre sur des échantillons tests provenant de patients pour lesquels un traitement antibiotique a été réalisé dans les 48 heures précédant le prélèvement desdits échantillons tests sanguins et éventuellement céphalo-rachidien.

On entend par traitement antibiotique, l'administration d'un antibiotique choisi dans le groupe comprenant la famille des β-lactamines comme la pénicilline et l'ampicilline, les céphalosporines la famille des aminosides comme la gentamicine, la famille des phénicolés comme le chloramphénicol.

En général, dans la pratique, une étape de coloration de Gram de l'échantillon de liquide céphalo-rachidien test permettant d'identifier la présence de bactéries dans ledit échantillon test est réalisée. La coloration de Gram du liquide céphalo-rachidien peut s'effectuer selon des techniques bien connues de l'Homme du Métier telle que la technique de Gram modifiée par Hucker. La coloration de Gram est une coloration différentielle basée sur la perméabilité plus grande de la paroi des bactéries à Gram négatif à l'alcool. Elle consiste, dans un premier temps, à réaliser un complexe colorant soluble dans l'alcool (cristal violet-lugol) qui colore en violet le cytoplasme de toutes les bactéries. Dans un second temps, intervient la décoloration par l'alcool, qui traverse bien la paroi des bactéries à Gram négatif, et qui dissout le complexe colorant. Chez les bactéries à Gram positif la paroi ne se laisse pas traverser. La coloration de Gram du liquide céphalo-rachidien peut permettre la détection de bactéries provoquant des méningites comme *H. influenzae, S. pneumoniae, N. meningitidis. H. influenzae* et *N. meningitidis* sont des bactéries Gram négatif tandis que *S. pneumoniae* est une bactérie Gram positif.

Le procédé selon l'invention est mis en oeuvre après une première étape de prélèvement d'un échantillon sanguin dudit individu et, éventuellement, d'un échantillon de liquide céphalo-rachidien dudit individu.

Les échantillons sanguins et du liquide céphalo-rachidien peuvent être obtenus simplement selon des techniques de prélèvement décrites précédemment.

Selon un mode de réalisation préféré de la méthode selon l'invention, celui-ci comprend en outre une étape d'administration d'antibiotiques aux patients pour lesquels la présence d'une méningite bactérienne a été diagnostiquée.

Un second objet de l'invention correspond à un kit consistant en :
- des moyens de détection de la procalcitonine
- des moyens de détection des protéines, de préférence dans un échantillon de liquide céphalo-rachidien.

Le kit selon l'invention permet de diagnostiquer la présence d'une méningite bactérienne chez un individu.

On entend par moyens de détection de la procalcitonine, des moyens de mise en oeuvre de techniques immunologiques quantitatives comprenant des anticorps ou des fragment d'anticorps qui lient spécifiquement la protéine procalcitonine. De tels moyens de détection permettent de déterminer la concentration de procalcitonine dans un échantillon sanguin.

Selon un mode de réalisation préféré du kit selon l'invention, lesdits moyens de détection de la procalcitonine sont des anticorps ou des fragments d'anticorps qui lient spécifiquement la procalcitonine.

On entend par anticorps aussi bien des anticorps polyclonaux que monoclonaux, de préférence des anticorps monoclonaux.

À titre d'exemples d'anticorps monoclonaux qui lient spécifiquement la procalcitonine, on peut citer les anticorps décrits dans les brevets US 6,451,311, US 5,330,909 et US 6,133,427, les anticorps disponibles chez ABCAM avec les références « ab14813 », « abc1498 », « abc1494 », « ab14817 », « ab14816 », « ab24454 », l'anticorps disponible chez CHEMICON avec la référence « MAB3490 » et les anticorps disponibles chez GeneTex®, Inc. avec les références « GTX14813 », « GTX11498 », « GTX11494 », « GTX14817 » et « GTX14816 ».

On entend par fragment d'anticorps qui lient spécifiquement la procalcitonine, les fragments Fab, F(ab')2, FV et sFv d'anticorps monoclonaux qui lient spécifiquement la procalcitonine.

Avantageusement, le kit selon l'invention comprend en outre une solution de procalcitonine à une concentration déterminée à titre d'échantillon de référence.

On entend par solution de procalcitonine à une concentration déterminée, une solution de procalcitonine à une concentration comprise entre 0,05 ng/mL et 10 ng/mL, de préférence entre 0,1 ng/mL et 1 ng/mL et préférentiellement de 0,5 ng/ml.

On entend par moyens permettant la détection des protéines dans un échantillon de liquide céphalo-rachidien, tout moyen permettant la révélation de la présence des protéines dans un échantillon de liquide céphalo-rachidien comme des anticorps dirigés contre des protéines du liquide céphalo-rachidien, des enzymes permettant de révéler l'activité desdites protéines ou encore des réactifs dont la réaction avec lesdites protéines permet de déterminer leur concentration comme des colorants. De tels moyens permettent de déterminer la concentration de protéines dans un échantillon de liquide céphalo-rachidien.

Selon un mode de réalisation préféré du kit selon l'invention, lesdits moyens permettant la détection des protéines dans un échantillon de liquide céphalo-rachidien sont des réactifs colorimétriques, dont la réaction avec lesdites protéines permet de déterminer la concentration en protéines dans ledit échantillon. À titre d'exemple de tels réactifs colorimétriques, on peut citer le rouge de pyrogallol, une solution d'amidoschwartz, une solution de méthanol acétique ou un mélange de ceux-ci.

Avantageusement encore, le kit selon l'invention comprend en outre une solution de protéines à une concentration déterminée.

On entend par solution de protéines à une concentration déterminée, une solution de protéines à une concentration comprise entre 0,05 et 5 g/L, de préférence entre 0,1 et 1 g/L et de manière particulièrement préférée de 0,5 g/L.

En général, une liste d'instructions à suivre afin d'utiliser lesdits moyens de détection pour diagnostiquer la présence d'une méningite bactérienne chez un individu est ajoutée au kit selon l'invention.

Un troisième objet de l'invention correspond à l'utilisation d'un kit tel que décrit précédemment pour la fabrication d'un outil de diagnostic de la méningite bactérienne.

Selon un mode de réalisation préféré de l'utilisation selon l'invention, ledit outil de diagnostic est destiné à des individus dont l'âge est compris entre 2 semaines et 25 ans, de préférence entre 3 semaines et 18 ans et tout préférentiellement entre 1 mois et 16 ans.

Selon un second mode de réalisation préféré de l'utilisation selon l'invention, ledit outil de diagnostic est destiné à des individus dont il est établi qu'ils sont atteints d'une méningite, laquelle est donc potentiellement d'origine bactérienne.

À titre d'exemple de tels individus, on peut citer des individus dont un échantillon de liquide céphalo-rachidien présente plus de 5 globules blancs/mm³ , de préférence plus de 7 globules blancs/mm³.

Selon un troisième mode de réalisation préféré de l'utilisation selon l'invention, ledit outil de diagnostic n'est pas destiné à des individus dont la probabilité d'atteinte par la méningite bactérienne est telle, que la mise en oeuvre dudit outil de diagnostic est inutile.

À titre d'exemple de tels individus, on peut citer les individus ayant subi préalablement une intervention neurochirurgicale, les individus souffrant d'immunodépression et/ou les individus présentant un purpura, un aspect septique et/ou toxique et/ou des convulsions.

Selon un quatrième mode de réalisation préféré de l'utilisation selon l'invention, ledit outil de diagnostic n'est pas destiné à des individus pour lesquels un traitement antibiotique a été réalisé dans les 48 heures précédant le prélèvement chez lesdits individus d'un échantillon sanguin et, éventuellement d'un échantillon de liquide céphalo-rachidien.

Selon un cinquième mode de réalisation préféré de l'utilisation selon l'invention, ledit outil de diagnostic n'est pas destiné à des individus présentant plus de 12 000 globules rouges/mm³, de préférence plus de 10 000 globules rouges/mm³ dans leur liquide céphalo-rachidien.

Un quatrième objet de l'invention correspond à l'utilisation d'un kit selon l'invention dans un procédé pour détecter la présence d'un méningite bactérienne.

Il ressort donc de l'invention qu'il est possible d'identifier la présence d'une méningite bactérienne en comparant la concentration en procalcitonine présente dans un échantillon sanguin test provenant d'un individu avec la concentration de procalcitonine présente dans un échantillon de référence ou avec une valeur de référence, et la concentration en protéines présentes dans un échantillon de liquide céphalo-rachidien test provenant d'un individu avec la concentration en protéines présentes dans un échantillon de référence ou avec une valeur de référence.

Avantageusement, lesdites comparaisons sont effectuées par des moyens informatiques, de préférence par un ordinateur.

Ces comparaisons permettent d'établir un diagnostic au regard d'une méningite bactérienne pour ledit individu.

Avantageusement encore, la méthode selon l'invention peu comprendre l'impression d'un rapport.

Ledit procédé est mis en oeuvre sur un individu ayant un âge compris entre 2 semaines et 25 ans, de préférence entre 3 semaines et 18 ans et tout préférentiellement entre 1 mois et 16 ans.

Ledit procédé est mis en oeuvre sur un individu dont le liquide céphalo-rachidien peut présente plus de 5 globules blancs/mm³, de préférence plus de 7 globules blancs/mm³.

Ledit procédé n'est pas mis en oeuvre sur un individu dont la probabilité de méningite bactérienne est telle, que la mise en oeuvre dudit procédé est inutile.

À titre d'exemple de tels individus, on peut citer les individus ayant subi préalablement une intervention neurochirurgicale, les individus souffrant d'immunodépression et les individus présentant un purpura, un aspect septique et/ou toxique et/ou des convulsions.

Ledit procédé n'est pas mis en oeuvre sur un individu pour lequel un traitement antibiotique a été réalisé dans les 48 heures précédant le prélèvement d'un échantillon sanguin et, éventuellement d'un échantillon de liquide céphalo-rachidien.

Ledit procédé n'est pas mis en oeuvre avec des concentrations de procalcitonine et, éventuellement, de protéines dans le liquide céphalo-rachidien obtenues à partir d'échantillons dont la qualité est insuffisante.

À titre d'exemple de tels échantillons, on peut citer des échantillons de liquide céphalo-rachidien dont le prélèvement a entraîné une hémorragie localisée du patient. De tels échantillons de liquide céphalo-rachidien présentent alors une concentration de globules rouges supérieure à 12 000 globules rouges/mm³, de préférence supérieure à 10 000 globules rouges/mm³.

Les exemples qui suivent permettent d'illustrer l'invention et sont donnés à titre non limitatifs.

### EXEMPLES

### I. Exemple 1 : Détection de la présence d'une méningite bactérienne sur une population de 201 patients dont 21 sont atteints de méningite bactérienne

### I.1 Population étudiée

L'étude a été basée sur 201 enfants admis à l'hôpital Saint-Vincent de Paul à Paris entre 2000 et 2004 pour les patients atteints de méningite virale et entre 1995 et 2004 pour les patients atteints de méningite bactérienne. L'intérêt de cette étude réside notamment dans le fait qu'elle a été menée sur une large population de patients chez lesquels la présence d'une méningite a été clairement établie a posteriori, après la période de vaccination contre *Haemophilus influenza b* qui a débutée en 1994 comme décrit dans la publication de Dumonceaux et al. (Archives de Pédiatrie, 1999, vol. 6, pages 617-624).

L'application à ces 201 patients de différentes règles connues pour distinguer les méningites bactériennes des méningites virales comme décrites précédemment (règles de Jaeger *et al*., Bonsu *et al.,* Freedman *et al.,* Ooestenbrink *et al.)* n'a pas permis de détecter d'une manière rapide et simple les patients atteints de méningites bactériennes avec une sensibilité de 100%, une bonne spécificité et une bonne applicabilité clinique. Seule la règle de Nigrovic *et al*. a permis de détecter l'ensemble des patients atteints d'une méningite bactérienne avec une bonne spécificité et une bonne applicabilité clinique. Toutefois, l'application de cette règle de Nigrovic *et al.* à une large population de 890 patients a montré que la sensibilité de cette règle n'était pas de 100% pour les méningites bactériennes.

Au regard de l'art antérieur, les Inventeurs ont donc cherché à établir un procédé rapide et simple permettant de distinguer les méningites bactériennes des méningites virales avec une sensibilité de 100% pour les méningites bactériennes.

Parmi ces 201 enfants, seuls les patients dont l'âge était compris entre 28 jours et 16 ans ont été pris en considération dans cette étude pour tester l'efficacité du procédé de détection de la présence d'une méningite bactérienne selon l'invention.

En outre, parmi ces 201 patients, seuls les patients présentant plus de 7 globules blancs/mm³ dans leur liquide céphalo-rachidien, ont été pris en considération dans cette étude.

Parmi ces 201 patients, les individus dont la probabilité d'atteinte par la méningite bactérienne était telle que la mise en oeuvre du procédé selon l'invention est inutile n'ont pas été pris en considération dans cette étude. Ainsi les patients ayant subi une intervention neurochirurgicale, les individus souffrant d'immunodépression et les individus présentant un purpura, un aspect septique et/ou toxique ou encore des convulsions ont été exclus de cette étude.

De plus, parmi ces 201 patients, les individus dont la qualité des échantillons ne permettait pas la mise en oeuvre du procédé selon l'invention n'ont pas été pris en considération dans cette étude. Ainsi, les patients dont le prélèvement de liquide céphalo-rachidien a entraîné une hémorragie, ces patients présentant plus de 10 000 globules rouge/mm³, ont été exclus de cette étude.

De même, les patients pour lesquels un traitement antibiotique a été réalisé dans les 48 heures précédant le prélèvement des échantillons tests sanguins et éventuellement de liquide céphalo-rachidien ont été exclus de cette étude.

En conséquence, parmi ces 201 patients seuls 167 patients ont été utilisés pour tester l'efficacité de détection de la présence d'une méningite bactérienne selon l'invention.

Parmi ces 167 patients, sur lesquelles l'étude a été menée, 146 patients ont été diagnostiqués comme présentant une méningite virale et 21 patients ont été diagnostiqués comme présentant une méningite bactérienne. Parmi ces 21 patients, 11 ont été admis à l'hôpital Saint-Vincent de Paul à Paris entre 1995 et 1999 et 10 entre 2000 et 2004. Les pathogènes responsables de la méningite bactérienne chez ces 21 patients sont *Streptococcus pneumoniae* chez 10 patients, *Neisseria meningitidis* chez 9 patients, *Haemophilus influenza b* chez 1 patient et *Streptococcus* group B chez 1 patient.

### 1.2 Détermination de la concentration en procalcitonine dans un échantillon sanguin test provenant d'un patient

3 mL de sang ont été prélevés chez les 167 patients à l'aide d'une aiguille munie d'une seringue introduite dans une veine du pli du coude des patients.

Les échantillons de sang ont été centrifugés pendant 10 minutes à 3800 rpm à température ambiante (environ 20°C) puis le plasma a été récupéré à l'aide d'une pipette en plastique.

La concentration en procalcitonine dans ces échantillons a été déterminée en utilisant une technique immunoluminométrique à l'aide de l'ensemble LUMItest® PCT disponible chez BRAHMS Diagnostica et selon le protocole de dosage de la Procalcitonine fourni avec cet ensemble.

### 1.3 Détermination de la concentration en protéines dans un échantillon de liquide céphalo-rachidien test provenant d'un patient

2 mL de liquide céphalo-rachidien ont été prélevés chez les 167 patients à l'aide d'une ponction lombaire. Les échantillons ont ensuite rapidement été utilisés pour le dosage des protéines totales selon la méthode colorimétrique au rouge de pyrogallol en utilisant l'ensemble disponible chez Roche Diagnostics avec la référence « 03515826 » et selon le protocole fourni avec cet ensemble.

### I.4 Détermination de la présence d'une méningite bactérienne

La détermination de la présence d'une méningite bactérienne chez un patient a ensuite été réalisée en fonction de la concentration en procalcitonine dans leur sang et de la concentration en protéines dans leur liquide céphalo-rachidien ; ces concentrations ayant été obtenues selon les méthodes décrites respectivement dans les paragraphes précédents I.1 et I.2. Le nombre de patients ayant une concentration en procalcitonine dans leur sang supérieure ou égale à 0,5 ng/mL (PCT ≥ 0,5 ng/mL) et/ou une concentration en protéines dans leur liquide céphalo-rachidien supérieure ou égale à 0,5 g/L (CSF ≥ 0,5 g/L) a été déterminé.

| | Méningite bactérienne (n=21) | | Méningite virale (n=146) | |
|---|---|---|---|---|
| | n | % | n | % |
| PCT ≥ 0,5 ng/mL | 16 | 89 | 15 | 11 |
| PCT < 0,5 ng/mL | 2 | 11 | 119 | 89 |
| CSF ≥ 0,5 g/L | 18 | 86 | 31 | 22 |
| CSF < 0,5 g/L | 3 | 14 | 112 | 78 |
| PCT ≥ 0,5 ng/mL ou CSF ≥ 0,5g/L | 21 | 100 | 85 | 58 |

Les inventeurs ont ainsi montré que tous les patients atteints d'une méningite bactérienne présentaient soit une concentration en procalcitonine dans leur sang supérieure ou égale à 0,5 ng/mL (PCT ≥ 0,5 ng/mL) soit une concentration en protéines dans leur liquide céphalo-rachidien supérieure ou égale à 0,5 g/L (CSF ≥ 0,5 g/L).

De plus, les inventeurs ont démontré que la détermination chez un patient d'une concentration en procalcitonine dans le sang supérieure ou égale à 0,5 ng/mL ou une concentration en protéines dans le liquide céphalo-rachidien supérieure ou égale à 0,5 g/L permet de détecter tous les patients atteints de méningite bactérienne.

En effet, les deux patients avec une méningite bactérienne présentant une concentration en procalcitonine dans leur sang inférieure à 0,5 ng/mL ont une concentration en protéines dans leur liquide céphalo-rachidien supérieure ou égale à 0,5 g/L. Les trois patients avec une méningite bactérienne présentant une concentration en protéines dans leur liquide céphalo-rachidien inférieure à 0,5 g/L ont une concentration en procalcitonine dans leur sang supérieure à 0,5 ng/mL.

En conséquence, les Inventeurs ont démontré que la combinaison des deux paramètres biologiques, concentration en procalcitonine dans le sang et concentration en protéines dans le liquide céphalo-rachidien d'un individu, permet de détecter la présence d'une méningite bactérienne chez un patient de manière simple et rapide avec une sensibilité de 100%.

### II. Exemple 2 : Détection de la présence d'une méningite bactérienne sur une population de 202 patients dont 87 sont atteints de méningite bactérienne

L'efficacité du procédé de détection de la présence d'une méningite bactérienne selon l'invention a été testée sur une population de 202 patients dont 87 patients étaient atteints de méningite bactérienne. L'étude a été menée telle que décrit précédemment dans l'exemple 1. Le procédé selon l'invention a permis de détecter tous les patients atteints de méningite bactérienne.

## Revendications

1. Procédé *in vitro* de détection de la présence d'une méningite bactérienne consistant en:
i) la détermination de la concentration de procalcitonine présente dans un échantillon sanguin test ; et
ii) la comparaison de la concentration ainsi déterminée à la concentration de procalcitonine présente dans un échantillon de référence ou à une valeur de référence ; et
iii) la détermination de la concentration de protéines présentes dans un échantillon de liquide céphalorachidien test ; et
iv) la comparaison de la concentration ainsi déterminée à la concentration de protéines présentes dans un échantillon de référence ou à une valeur de référence.

2. Procédé selon la revendication 1, **caractérisé en ce que** ledit échantillon de référence de l'étape ii) est choisi dans le groupe comprenant :
- un échantillon sanguin provenant d'un individu sain;
- un échantillon sanguin provenant d'un individu chez lequel une méningite virale est présente ; et
- une solution de procalcitonine à une
concentration déterminée, notamment une concentration de procalcitonine comprise entre 0,05 ng/mL et 10 ng/mL de préférence entre 0,1 ng/mL et 1 ng/mL.

3. Procédé selon l'une quelconque des revendications 1 ou 2, **caractérisé en ce que** l'étape i) est effectuée par des techniques immunologiques quantitatives.

4. Procédé selon l'une quelconque des revendications 1 à 3, **caractérisé en ce que** ledit échantillon de référence à l'étape iv) est choisi dans le groupe comprenant :
- un échantillon de liquide céphalo-rachidien provenant d'un individu sain,
- un échantillon de liquide céphalo-rachidien provenant d'un individu chez lequel une méningite virale est présente ; et
- une solution de protéines à une concentration déterminée, notamment une concentration de protéines comprise entre 0,05 g/L et 5 g/L, de préférence entre 0,1 g/L et 1 g/L.

5. Procédé selon l'une quelconque des revendication 1 à 4, **caractérisé en ce que** l'étape iii) est effectuée par un test colorimétrique.

6. Procédé selon l'une quelconque des revendications 1 à 5, **caractérisé en ce que** ledit échantillon sanguin test et éventuellement ledit échantillon de liquide céphalo-rachidien test proviennent d'un individu dont l'âge est compris entre 2 semaines et 25 ans, de préférence entre 3 semaines et 18 ans.

7. Procédé selon l'une quelconque des revendications 1 à 6, **caractérisé en ce que** ledit échantillon sanguin test et éventuellement ledit échantillon de liquide céphalo-rachidien test proviennent d'un individu dont il est établi qu'il est atteint d'une méningite, notamment d'un individu présentant plus de 5 globules blancs/mm³, de préférence plus de 7 globules blancs/mm³ dans son liquide céphalo-rachidien.

8. Procédé selon l'une quelconque des revendications 1 à 7, **caractérisé en ce que** ledit échantillon sanguin test et éventuellement ledit échantillon de liquide céphalo-rachidien test ne provient pas d'un individu ayant subi préalablement une intervention neurochirurgicale, d'un individu souffrant d'immunodépression et/ou d'un individu présentant un purpura, un aspect septique et/ou toxique et/ou des convulsions.

9. Procédé selon l'une quelconque des revendications 1 à 8, **caractérisé en ce que** ledit échantillon sanguin test et éventuellement ledit échantillon de liquide céphalo-rachidien test ne provient pas d'un individu pour lequel un traitement antibiotique a été réalisé dans les 48 heures précédant le prélèvement dudit échantillon sanguin test et éventuellement dudit échantillon de liquide céphalo-rachidien test.

10. Procédé selon l'une quelconque des revendications 1 à 9, **caractérisé en ce que** ledit échantillon de liquide céphalo-rachidien test ne présente pas une concentration en globules rouges supérieure à 12 000 globules rouges/mm³, de préférence supérieure à 10 000 globules rouges/mm³.

11. Kit consistant en :
- des moyens de détection de la procalcitonine et éventuellement une solution de procalcitonine à une concentration déterminée ; et
- des moyens de détection des protéines dans un échantillon de liquide céphalo-rachidien et éventuellement une solution de protéines à une concentration déterminée.

12. Kit selon la revendication 11 **caractérisé en ce que** lesdits moyens de détection de la procalcitonine sont des moyens de mise en oeuvre de techniques immunologiques quantitatives comprenant des anticorps ou des fragments d'anticorps qui lient spécifiquement la procalcitonine.

13. Kit selon l'une des revendications 11 ou 12, **caractérisé en ce que** lesdits moyens de détection des protéines sont des réactifs colorimétriques dont la réaction avec lesdites protéines permet de déterminer leur concentration, lesdits moyens de détection étant choisis dans le groupe comprenant le rouge de pyrogallol, une solution d'amidoschwartz, une solution de méthanol acétique ou un mélange de ceux-ci.

14. Utilisation d'un Kit selon l'une quelconque des revendications 11 à 13 pour la fabrication d'un outil de diagnostic de la méningite bactérienne.

15. Utilisation selon la revendication 14 **caractérisée en ce que** ledit outil de diagnostic est destiné à des individus dont l'âge est compris entre 2 semaines et 25 ans, de préférence entre 3 semaines et 18 ans.

16. Utilisation selon l'une quelconque des revendications 14 ou 15 **caractérisée en ce que** ledit outil de diagnostic est destiné à des individus dont il est établi qu'ils sont atteints de méningite, notamment des individus présentant plus de 5 globules blancs/mm³, de préférence plus de 7 globules blancs/mm³ dans leur liquide céphalo-rachidien.

17. Utilisation selon l'une quelconque des revendications 14 à 16 **caractérisée en ce que** ledit outil de diagnostic n'est pas destiné à des individus ayant subi préalablement une intervention neurochirurgicale, des individus souffrant d'immunodépression et/ou des individus présentant un purpura, un aspect septique et/ou des convulsions.

18. Utilisation selon l'une quelconque des revendications 14 à 17 **caractérisée en ce que** ledit outil de diagnostic n'est pas destiné à des individus présentant plus de 12 000 globules rouges/mm³, de préférence plus de 10 000 globules rouges/mm³ dans leur liquide céphalo-rachidien.

19. Utilisation selon l'une quelconque des revendications 14 à 18 **caractérisée en ce que** ledit outil de diagnostic n'est pas destiné à des individus pour lesquels un traitement antibiotique a été réalisé dans les 48 heures précédant le prélèvement chez lesdits individus d'un échantillon sanguin et éventuellement d'un échantillon de liquide céphalo-rachidien.

20. Utilisation d'un Kit selon l'une quelconque des revendications 11 à 13 dans un procédé *in vitro* pour détecter la présence d'une méningite bactérienne chez un individu.

## Claims

1. Method for the *in vitro* detection of a bacterial meningitis consisting of:
i) determining the concentration of procalcitonine which is present in a blood sample to be tested; and
ii) comparing the thus determined concentration to the concentration of procalcitonine which is present in a standard sample or to a standard value; and
iii) determining the concentration of proteins present in a sample of cerebrospinal fluid to be tested; and
iv) comparing the thus determined concentration with the concentration of proteins present in a standard sample or to a standard value.

2. Method according to claim 1, **characterized in that** said standard sample in step ii) is selected within the group comprising:
- a blood sample from a healthy subject;
- a blood sample from a subject with viral meningitis; and
- a procalcitonine solution of predetermined concentration, especially a procalcitonine concentration from 0.05 ng/mL to 10 ng/mL, preferably 0.1 ng/mL to 1 ng/mL.

3. Method according to either one of claims 1 or 2, **characterized in that** step i) is performed using quantitative immunological techniques.

4. Method according to any one of claims 1-3, **characterized in that** said standard sample in step iv) is selected within the group comprising :
- a cerebrospinal fluid sample from a healthy subject,
- a cerebrospinal fluid sample from a subject with viral meningitis; and
- a solution of proteins at a predetermined concentration, especially a protein concentration from 0.05 g/L to 5 g/L, preferably 0.1 g/L to 1 g/L.

5. Method according to any one of claims 1-4, **characterized in that** step iii) is performed using a colorimetric test.

6. Method according to any one of claims 1-5, **characterized in that** said blood sample to be tested and optionally said cerebrospinal fluid sample to be tested have been taken from a subject aged 2 weeks to 25 years, preferably 3 weeks to 18 years.

7. Method according to any one of claims 1-6, **characterized in that** said blood sample to be tested and optionally said cerebrospinal fluid sample to be tested have been taken from a subject that is found to be suffering from meningitis, especially from a subject with more than 5 leucocytes/mm³, preferably with more than 7 leucocytes/mm³ in its cerebrospinal fluid.

8. Method according to any one of claims 1-7, **characterized in that** said blood sample to be tested and optionally said cerebrospinal fluid sample to be tested have not been taken from a subject who has previously undergone neurosurgery, an immunodepressed subject and/or a subject with purpura, septic and/or toxic symptoms and/or seizures.

9. Method according to any one of claims 1-8, **characterized in that** said blood sample to be tested and optionally said cerebrospinal fluid sample to be tested have not been taken from a subject for whom antibiotic treatment was carried out within 48 hours preceding the taking of said blood sample to be tested and optionally of said cerebrospinal fluid sample to be tested.

10. Method according to any one of claims 1-9, **characterized in that** said cerebrospinal fluid sample to be tested does not contain a red blood cell concentration above 12,000 red blood cells/mm³, preferably above 10,000 red blood cells/mm³.

11. Kit consisting of:
- means for detecting procalcitonine and optionally a procalcitonine solution of predetermined concentration; and
- means for detecting proteins in a cerebrospinal fluid sample and optionally a protein solution of predetermined concentration.

12. Kit according to claim 11, **characterized in that** said means for detecting procalcitonine are means implementing quantitative immunological techniques comprising antibodies or antibody fragments which specifically bind to procalcitonine.

13. Kit according to either one of claims 11 or 12, **characterized in that** said means for detecting proteins are colorimetric reagents, that react with said proteins to determine their concentration, said detection means being selected within the group comprising pyrogallol red, a solution of amidoschwartz, a solution of acetic methanol or a mixture thereof.

14. Use of a Kit according to any one of claims 11-13 for the manufacturing of a diagnostic tool for bacterial meningitis.

15. Use according to claim 14, **characterized in that** said diagnostic tool is intended for subjects aged 2 weeks to 25 years, preferably 3 weeks to 18 years.

16. Use according to either one of claims 14 or 15, **characterized in that** said diagnostic tool is intended for subjects that are found to be suffering from meningitis, especially subjects with more than 5 leucocytes/mm³, preferably more than 7 leucocytes/mm³ in their cerebrospinal fluid.

17. Use according to any one of claims 14-16, **characterized in that** said diagnostic tool is not intended for subjects having previously undergone neurosurgery, immunodepressed subjects and/or subjects with purpura, septic and/or toxic symptoms and/or seizures.

18. Use according to any one of claims 14-17, **characterized in that** said diagnostic tool is not intended for subjects with more than 12,000 red blood cells/mm³, preferably with more than 10,000 red blood cells/mm³ in their cerebrospinal fluid.

19. Use according to any one of claims 14-18, **characterized in that** said diagnostic tool is not intended for subjects for whom antibiotic treatment was carried out within 48 hours preceding the taking in said subjects of a blood sample to be tested and optionally of a cerebrospinal fluid sample to be tested.

20. Use of a kit according to any one of claims 14-19 in an *in vitro* method for detecting the presence of bacterial meningitis in a subject.

## Patentansprüche

1. In Vitro-Verfahren zur Feststellung des Vorhandenseins einer bakteriellen Meningitis, bestehend in:
i) der Bestimmung der in einer Test-Blutprobe vorhandenen Konzentration an Procalcitonin; und
ii) dem Vergleich der somit bestimmten Konzentration an Procalcitonin mit der in einem Referenzmuster vorhandenen Konzentration an Procalcitonin oder einem Referenzwert; und
iii) der Bestimmung der in einer flüssigen zerebrospinalen Probe vorhandenen Konzentration an Proteinen; und
iv) dem Vergleich der somit bestimmten Konzentration mit der in einer Referenzprobe vorhandenen Konzentration an Proteinen oder einem Referenzwert.

2. Verfahren gemäß Anspruch 1, **dadurch gekennzeichnet, dass** die genannte Referenzprobe der Stufe ii) aus der Gruppe ausgewählt ist, die Folgendes umfasst:
- eine aus einem gesunden Patienten stammende Blutprobe;
- eine aus einem Patienten stammende Blutprobe, bei dem eine virale Meningitis vorhanden ist; und
- eine Procaclitoninlösung in einer bestimmten Konzentration, insbesondere einer Konzentration an Procalcitonin, die zwischen 0,05 ng/ml und 10 ng/ml inbegriffen ist, bevorzugt zwischen 0,1 ng/ml und 1 ng/ml.

3. Verfahren gemäß Anspruch 1 oder 2, **dadurch gekennzeichnet, dass** die Stufe i) durch quantitative immunologische Techniken durchgeführt wird.

4. Verfahren gemäß Anspruch 1 bis 3, **dadurch gekennzeichnet, dass** die genannte Referenzprobe in Stufe iv) aus der Gruppe ausgewählt ist, die Folgendes umfasst:
- eine aus einem gesunden Patienten stammende flüssige zerebrospinale Probe,
- eine aus einem Patienten, bei dem eine virale Meningitis vorhanden ist, stammende flüssige zerebrospinale Probe; und
- eine Proteinlösung in einer bestimmten Konzentration, insbesondere einer Konzentration an Proteinen, die zwischen 0,05 g/l und 5 g/l inbegriffen ist, bevorzugt zwischen 0,1 g/l und 1 g/l.

5. Verfahren gemäß Anspruch 1 bis 4, **dadurch gekennzeichnet, dass** die Stufe iii) durch einen kolorimetrischen Test durchgeführt wird.

6. Verfahren gemäß Anspruch 1 bis 5, **dadurch gekennzeichnet, dass** die genannte Testblutprobe und eventuell die genannte zerebrospinale, flüssige Testprobe aus einem Patienten stammen, dessen Alter zwischen 2 Wochen und 25 Jahren inbegriffen ist, bevorzugt zwischen 3 Wochen und 18 Jahren.

7. Verfahren gemäß Anspruch 1 bis 6, **dadurch gekennzeichnet, dass** die genannte Testblutprobe und eventuell die genannte zerebrospinale flüssige Testprobe aus einem Patienten stammen, von dem feststeht, dass er unter einer Meningitis leidet, insbesondere von einem Patienten, der mehr als 5 weiße Blutkörperchen / mm³, bevorzugt mehr als 7 weiße Blutkörperchen / mm³ in seiner zerebrospinalen Flüssigkeit aufweist.

8. Verfahren gemäß Anspruch 1 bis 7, **dadurch gekennzeichnet, dass** die genannte Testblutprobe und eventuell die genannte zerebrospinale flüssige Testprobe nicht aus einem Patienten stammen, der zuvor einem neurochirurgischen Eingriff unterzogen wurde, einem Patienten, der an einer Immundepression leidet und / oder einem Patienten, der eine Purpura, einen septischen und / oder toxischen Aspekt und / oder Krämpfe aufweist.

9. Verfahren gemäß Anspruch 1 bis 8, **dadurch gekennzeichnet, dass** die genannte Testblutprobe und eventuell die genannte zerebrospinale flüssige Testprobe nicht aus einem Patienten stammen, für den eine Behandlung mit Antibiotika in den 48 Stunden vor der Entnahme der genannten Testblutprobe und eventuell der genannten zerebrospinalen flüssigen Testprobe realisiert wurde.

10. Verfahren gemäß Anspruch 1 bis 9, **dadurch gekennzeichnet, dass** die genannte zerebrospinale flüssige Testprobe keine Konzentration an roten Blutkörperchen von mehr als 12.000 roten Blutkörperchen / mm³, bevorzugt mehr als 10.000 roten Blutkörperchen / mm³ aufweist.

11. Kit bestehend aus:
- Feststellungsmitteln des Procalcitonins und eventuell einer Procalcitoninlösung in einer bestimmten Konzentration; und
- Feststellungsmittel der Proteine in einer zerebrospinalen flüssigen Probe und eventuell einer Proteinlösung in einer bestimmten Konzentration.

12. Kit gemäß Anspruch 11, **dadurch gekennzeichnet, dass** die genannten Feststellungsmittel des Procalcitonins Mittel zur Umsetzung von quantitativen immunologischen Techniken sind, die Antikörper oder Antikörperfragmente umfassen, die spezifisch das Procalcitonin binden.

13. Kit gemäß Anspruch 11 oder 12, **dadurch gekennzeichnet, dass** die genannten Erfassungsmittel der Proteine kolorimetrische Reagenzien sind, deren Reaktion mit den genannten Proteinen die Bestimmung ihrer Konzentration zulässt, wobei die genannten Feststellungsmittel aus der Gruppe ausgewählt sind, die Pyrogallolrot, eine Amidoschwarzlösung, eine essigsaure Methanollösung oder eine Mischen aus denselben umfasst.

14. Verwendung eines Kits gemäß Anspruch 11 bis 13 für die Herstellung eines Diagnoseinstruments der bakteriellen Meningitis.

15. Verwendung gemäß Anspruch 14, **dadurch gekennzeichnet, dass** das genannte Diagnoseinstrument für Patienten bestimmt ist, deren Alter zwischen 2 Wochen und 25 Jahren, bevorzugt zwischen 3 Wochen und 18 Jahren inbegriffen ist.

16. Verwendung gemäß Anspruch 14 oder 15, **dadurch gekennzeichnet, dass** das genannte Diagnoseinstrument für Patienten bestimmt ist, von denen feststeht, dass sie unter Meningitis leiden, insbesondere für Patienten, die mehr als 5 weiße Blutkörperchen / mm³, bevorzugt mehr als 7 weiße Blutkörperchen / mm³ in ihrer zerebrospinalen Flüssigkeit aufweisen.

17. Verwendung gemäß Anspruch 14 bis 16, **dadurch gekennzeichnet, dass** das genannten Diagnoseinstrument nicht für Patienten bestimmt ist, die zuvor einem neurochirurgischen Eingriff unterzogen wurden, für Patienten, die an einer Immundepression leiden und / oder für Patienten, die eine Purpura, einen septischen Aspekt und / oder Krämpfe aufweisen.

18. Verwendung gemäß Anspruch 14 bis 17, **dadurch gekennzeichnet, dass** das genannte Diagnoseinstrument nicht für Patienten bestimmt ist, die mehr als 12.000 rote Blutkörperchen / mm³, bevorzugt mehr als 10.000 rote Blutkörperchen /mm³ in ihrer zerebrospinalen Flüssigkeit aufweisen.

19. Verwendung gemäß Anspruch 14 bis 18, **dadurch gekennzeichnet, dass** das genannte Diagnoseinstrument nicht für Patienten bestimmt ist, für die eine Behandlung mit Antibiotika in den 48 Stunden vor der Entnahme einer Blutprobe und eventuell einer zerebrospinalen flüssigen Probe bei den genannten Patienten realisiert wurde.

20. Verwendung eines Kits gemäß Anspruch 11 bis 13 in einem In Vitro-Verfahren zur Feststellung des Vorhandenseins einer bakteriellen Meningitis bei einem Patienten.
